# EUROPÄISCHE PATENTSCHRIFT

(11) **EP 0 696 570 B1**
(45) Veröffentlichungstag und Bekanntmachung des Hinweises auf die Patenterteilung: **14.01.1998**
(21) Anmeldenummer: 95111993.2
(22) Anmeldetag: 31.07.1995
(51) Int. Cl.: C07C 205/06, C07C 201/08

(54) **Verfahren zur Herstellung von Dinitrotoluol**
Process for the preparation of dinitrotoluene
Procédé de préparation de dinitrotoluène

(30) Priorität: 11.08.1994 DE 4428459
(43) Veröffentlichungstag der Anmeldung: 14.02.1996
(73) Patentinhaber: BAYER AG, 51368 Leverkusen (DE)
(72) Erfinder: Klingler, Uwe, D-41539 Dormagen (DE); Schieb, Thomas, Dr., D-51503 Rösrath (DE); Wiechers, Gerhard, Dr., D-51381 Leverkusen (DE); Zimmermann, Jürgen, Dr., Walnut Creek, CA 94598 (DE)

(56) Entgegenhaltungen:
- EP-A- 0 155 586
- EP-A- 0 597 361
- DE-A- 2 655 197

## Beschreibung

Die vorliegende Erfindung betrifft ein Verfahren zur zweistufigen Herstellung von Dinitrotoluol aus Toluol und Salpetersäure in Gegenwart von Schwefelsäure.

Dinitrotoluol (DNT) ist eine Vorstufe zur Herstellung von Toluylendiisocyanat (TDI). TDI ist ein Ausgangsprodukt für Polyurethankunststoffe. DNT wird auf herkömmlichem Wege durch Umsetzung von Toluol mit Nitriersäure erhalten. Die Reaktion wird in zwei Stufen durchgeführt. Zunächst wird durch Umsetzung von Toluol mit einer verdünnten Nitriersäure Mononitrotoluol (MNT) erhalten. Nach Abtrennung der gebrauchten Schwefelsäure (im folgenden "Absäure" genannt) wird dieses MNT in einem zweiten Verfahrensschritt in DNT überführt. Beide Nitrierschritte werden isotherm, d.h. unter Kühlung bei gleichbleibend niedriger Temperatur durchgeführt.

Im Verfahren fallen zwei Absäuren an, die nach Aufstockung mit Salpetersäure wieder zurückgeführt werden können. Die Rückführung ist bei der Absäure aus der zweiten Stufe direkt möglich. Die Absäure ist noch so konzentriert, daß sie nach Aufstockung mit Salpetersäure ohne vorherige Aufkonzentrierung in der 1. Stufe eingesetzt werden kann. Aufkonzentriert werden muß dann nur die Absäure aus der 1. Stufe. Dieses erfolgt üblicherweise nach dem Pauling-Verfahren (Bodenbrenner, von Plessen, Vollmüller, Dechema-Monogr. 86 (1980), 197) oder in jüngerer Zeit vermehrt auch nach dem Vakuumverfahren (Winnacker, Küchler, Chem. Technol., Bd. 2, Anorg. Technol. I, 4. Aufl., 1982, S. 70 bis 72). Beiden Verfahren ist gemeinsam, daß Energie zum Austreiben des Reaktionswassers eingebracht werden muß.

Bei der Rückführung der aufkonzentrierten Absäure wird diese mit hochkonzentrierter Salpetersäure aufgestockt. Ein Einsatz von niederkonzentrierten Salpetersäuren, die deutlich billiger sind, ist zwar prinzipiell möglich, ist jedoch mit einem erheblichen zusätzlichen Energieaufwand verbunden, da dann die Absäure höher aufkonzentriert werden muß.

Aus den genannten Gründen sind schon seit Jahren Bestrebungen im Gange, die Nitrierverfahren in den angeführten Schwachpunkten zu verbessern.

Durch adiabatische Reaktionsführung bei der Mononitrierung von Benzol gelang eine Verbesserung in energetischer Hinsicht, und es war möglich, verdünntere Salpetersäuren einzusetzen. Dieses Verfahren wird mittlerweile auch großtechnisch ausgeübt (US 3 928 475, US 4 021 498, US 4 091 042, US 4 453 027, EP-A 436 443).

Das adiabatische Verfahren konnte auch auf die Herstellung von Dinitroaromaten angewendet werden (EP-A 597 361). Hier wird Toluol einstufig mit Nitriersäure zu DNT umgesetzt. Durch Verwendung von Nitriersäuren spezieller Zusammensetzung ist es möglich, den Nitrierprozeß adiabatisch zu betreiben und die Reaktionswärme im System zu halten. Ein Kühlen des Prozesses wie beim herkömmlichen isothermen Verfahren ist nicht mehr erforderlich, wodurch teure Kühlleistung eingespart wird. Nach Trennung der Phasen wird die heiße Absäure im Vakuum verdüst und die Reaktionswärme des Prozesses zur Aufkonzentrierung der Absäure genutzt. Aufgrund der adiabatischen Reaktionsführung und der damit verbundenen hohen Reaktionstemperatur verträgt der Prozeß den Einsatz von verdünnter Salpetersäure. Je nach eingesetzter Salpetersäure ist kein bzw. nur noch ein geringes Aufheizen im Aufkonzentrierschritt erforderlich.

Von Nachteil bei diesem Verfahren ist, daß sowohl Mono- als auch Dinitrierung mit der gleichen Nitriersäure ausgeführt werden. Diese muß, da im Prozeß quantitativ nitriert werden soll, zwangsweise in ihrer Reaktivität auf den Dinitrierungsschritt abgestimmt werden. Für die Mononitrierung ist die verwendete Nitriersäure daher eigentlich zu konzentriert, so daß der Eintritt der ersten Nitrogruppe sehr schnell und gegebenenfalls auch weniger selektiv abläuft. Auch für die Aufkonzentrierung der Absäure ergeben sich Konsequenzen, nämlich daß im Mononitrierungsschritt eine konzentriertere Absäure anfällt als erforderlich. Das Aufkonzentrieren höherkonzentrierter Absäuren ist energetisch deutlich aufwendiger, da die Lösungswärme von Wasser in Schwefelsäure mit steigender Schwefelsäurekonzentration ansteigt.

Weiterhin nachteilig bei dem Verfahren ist die Tatsache, daß im Aufkonzentrierschritt in der Absäure gelöstes DNT mit überdestilliert wird. Unter den für die Kondensation des Wassers notwendigen Bedingungen kristallisiert dieses aus, belegt den Wärmetauscher und behindert somit den Wärmeübergang.

Als Möglichkeit zur Lösung des Problems bieten sich getaktete Wärmetauscher an. Diese werden jeweils wechselseitig betrieben und nach Belegung in der Ruhephase abgeschmolzen. Problematisch bei der Belegung der Austauscherfläche ist die dadurch rasch nachlassende Kühlleistung. Dementsprechend muß häufig getaktet werden. Das Abschmelzen des jeweils ruhenden Kondensators erfordert zusätzliche Energie.

Als weitere Möglichkeit zur Kondensation von feststoffbildenden Brüden wird der Misch- oder Einspritzkondensator beschrieben (R.A. Vauck, H.A. Müller, Grundoperationen chemischer Verfahrenstechnik, 5. Auflage, VEB Leipzip 1962, S. 447). Darunter wird das Einbringen der Brüdengase in einen Sprühstrahl aus kaltem Wasser verstanden, wobei für den obengenannten Fall DNT in feinverteilter fester Form abgeschieden wird. Aufgrund der hierfür notwendigen großen Wassermengen wird das Wasser sinnvollerweise im Kreis geführt und im Rückführungszweig gekühlt. Dabei besteht allerdings die Gefahr der Verstopfung von Leitungen und Düsen durch organische Komponenten mit niedrigem Schmelzpunkt, die zum Verkleben neigen.

Eine elegante Lösung zur Umgehung dieser Probleme ist durch das isotherme, zweistufige Nitrierverfahren gefunden worden. Dort wird MNT aus der ersten Nitrierstufe in die Brüden der zweiten Aufkonzentrierung eingespritzt. Das zugegebene MNT führt zu einer Schmelzpunkterniedrigung des DNT-Isomerengemisches und das kondensierte Stoffgemisch bleibt flüssig (DE-A 3 409 719).

Aufgabe war es, ein Verfahren zur Herstellung von Dinitrotoluol zur Verfügung zu stellen, welches den Einsatz von verdünnter Salpetersäure zuläßt, die Reaktionswärme nutzt und Probleme bei der Kondensation der Brüden im Aufkonzentrierschritt vermeidet.

Diese Aufgabe konnte durch das erfindungsgemäße Verfahren gelöst werden.

Gegenstand der Erfindung ist ein zweistufiges Verfahren zur kontinuierlichen Herstellung von Dinitrotoluol-Isomerengemischen durch Nitrierung von Toluol, welches dadurch gekennzeichnet ist, daß man in der ersten Stufe Toluol und Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 Gew.-% aus Nitrotoluol-Isomeren und zum Rest aus Nebenprodukten bestehen, in einem kontinuierlich betriebenen Reaktor adiabatisch unter Einstellung eines Molverhältnisses von Salpetersäure zu Toluol von mindestens 0,7:1 und höchstens 1,2:1 bei Temperaturen von 0 bis 140°C, vorzugsweise von 20 bis 140°C zur Reaktion bringt, anschließend die Phasen trennt und die Säurephase durch Entspannungsverdampfung gegebenenfalls unter gleichzeitiger Wärmezufuhr von mindestens 5 % des Wassers destillativ befreit und nach Zugabe von 50 bis 100 Gew.-%iger Salpetersäure kontinuierlich in die Reaktion zurückführt, die in der ersten Stufe abgetrennte organische Phase (hauptsächlich Mononitrotoluol; MNT) unter adiabatischen Bedingungen bei Temperaturen von 20 bis 200°C, vorzugsweise 40 bis 180°C, besonders bevorzugt von 60 bis 170°C, mit einer Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 Gew.-% aus Nitrotoluol-Isomeren und zum Rest aus Nebenprodukten bestehen, zur Reaktion bringt, wobei das Molverhältnis von Salpetersäure zu Mononitrotoluol mindestens 0,7:1 und höchstens 1,2:1 beträgt, anschließend die Phasen trennt und die Säurephase aus der zweiten Stufe durch Entspannungsverdampfung gegebenenfalls unter gleichzeitiger Wärmezufuhr von mindestens 5 % des Wassers destillativ befreit und nach Zugabe von 50 bis 100 Gew.-%iger Salpetersäure kontinuierlich in die Reaktion zurückführt und den Brüden bei der Aufkonzentrierung der zweiten Absäure Mononitrotoluol aus der ersten Stufe zuführt.

Die Menge an zugesetztem MNT wird so gewählt, daß das Brüdenkondensat flüssig abläuft und keine festen Beläge bildet. Dies ist der Fall, wenn in der organischen Phase des Brüdenkondensates ein Gewichtsverhältnis von MNT zu DNT von 2:1 bis 10:1 vorliegt. Die organischen Bestandteile des Brüdenkondensates werden nach der Phasentrennung in die erste und/oder zweite Nitrierstufe zurückgeführt.

Bevorzugt wird das erfindungsgemäße Verfahren mit zwei unterschiedlich konzentrierten Nitriersäuren betrieben. In der ersten Stufe wird vorzugsweise eine niedrig konzentrierte Nitriersäure verwendet, so daß eine selektive Nitrierung und eine energieoptimierte Aufkonzentrierung der Absäure möglich ist. In der zweiten Stufe kommt aufgrund des höheren Nitrierbedarfs eine konzentrierte Nitriersäure zur Anwendung.

Die Erfindung soll anhand der nachfolgenden Beispiele näher erläutert werden.

### Beispiel 1

Die verwendeten Reaktoren bestehen aus einer Vermischungsdüse (Innendurchmesser 0,2 mm) und zwei Redispergierdüsen (Innendurchmesser 0,3 mm). Zwischen den einzelnen Düsen befinden sich Verweilzeitstrecken (Innendurchmesser 2,0 mm), die für die jeweilige Umsetzung optimiert sind.

100,5 g/h (1,090 mol/h) Toluol und 2500 g/h (1,179 mol/h) einer Nitriersäure der Zusammensetzung 72,4:3,0:24,6 (Gew.-% H₂SO₄:HNO₃:H₂O) werden bei 27°C kontinuierlich in dem oben beschriebenen Reaktor unter adiabatischen Bedingungen zur Reaktion gebracht. Das den Reaktor verlassende Reaktionsgemisch wird bei der sich einstellenden Temperatur (ca. 50°C) einer Phasentrennung unterworfen. Während die Absäure eine Aufkonzentrierung durchläuft und nach Aufstockung mit 60 %iger Salpetersäure in die erste Reaktionsstufe zurückgeführt wird, wird die abgetrennte organische Phase bei der gleichen Temperatur (ca. 50°C) in einem zweiten Reaktor mit 2038,8 g/h (1,168 mol/h) einer Nitriersäure der Zusammensetzung 79,9:3,6:16,5 (Gew.-% H₂SO₄:HNO₃:H₂O) unter ebenfalls adiabatischen Bedingungen umgesetzt. Anschließend wird eine Phasentrennung durchgeführt. Die Absäure durchläuft nach Abtrennung eine Aufkonzentrierung. Um Ablagerungen im Kondensationsteil zu vermeiden, werden 15 g/h MNT aus der ersten Nitrierstufe in die überhitzten Brüden des Eindampfers gegeben. Die aufkonzentrierte Absäure wird nach Aufstockung mit 60 %iger Salpetersäure in die zweite Nitrierstufe zurückgeführt, ebenso die organischen Bestandteile des Brüdenkondensates. Die organische Phase wird wie üblich gewaschen (Wasser- und Sodawäsche) und liefert bei geschlossenen Kreisläufen 196,8 g/h Dinitrotoluol (99,0 %).

### Beispiel 2

101,2 g/h (1,102 mol/h) Toluol und 2500 ml/h (1,1903 mol/h) einer Nitriersäure der Zusammensetzung 68,5:3,0:28,5 (Gew.-% H₂SO₄:HNO₃:H₂O) werden bei 85°C kontinuierlich in dem in Beispiel 1 beschriebenen Reaktor unter adiabatischen Bedingungen zur Reaktion gebracht. Das austretende Reaktionsprodukt wird bei der sich einstellenden Temperatur in zwei Phasen aufgetrennt. Während die Absäure einer Aufkonzentrierung zugeführt und nach Aufstockung mit 60 %iger Salpetersäure in die erste Reaktionsstufe zurückgeführt wird, wird die organische Phase mit 2072,9 g/h (1,183 mol/h) einer Nitriersäure der Zusammensetzung 77,9:3,6:18,5 (Gew.-% H₂SO₄:HNO₃:H₂O) in einem zweiten Reaktor unter adiabatischen Bedingungen umgesetzt. Die Starttemperatur dieser Reaktion entspricht der Temperatur, die die rückgeführte aufkonzentrierte Absäure aus der gleichen Stufe nach Aufkonzentrierung und Aufstockung mit frischer Salpetersäure hat. Anschließend erfolgt eine Phasentrennung zur Isolierung des Dinitrotoluols. Dieses wird wie üblich mit Wasser und Soda gewaschen. Die erhaltene Absäure wird aufkonzentriert. Um Ablagerungen im Kondensationsteil zu vermeiden, werden 8g/h MNT aus der ersten Nitrierstufe in die überhitzten Brüden des Eindampfers gegeben. Die aufkonzentrierte Absäure wird nach Aufstrockung mit 60 %iger Salpetersäure in die zweite Nitrierstufe zurückgeführt, ebenso die organischen Bestandteile des Brüdenkondensats. Im kontinuierlich betriebenen Zustand mit geschlossenen Kreisläufen beträgt die Ausbeute an Dinitrotoluol 197,6 g/h (98,7 %).

## Patentansprüche

1. Zweistufiges Verfahren zur kontinuierlichen Herstellung von Dinitrotoluol-Isomerengemischen durch Nitrierung von Toluol, dadurch gekennzeichnet, daß man in der ersten Stufe Toluol und Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 Gew.-% aus Nitrotoluol-Isomeren und zum Rest aus Nebenprodukten bestehen, in einem kontinuierlich beschriebenen Reaktor adiabatisch unter Einstellung eines Molverhältnisses von Salpetersäure zu Toluol mindestens 0,7:1 und höchstens 1,2:1 bei Temperaturen von 0 bis 140°C, vorzugsweise von 20 bis 140°C zur Reaktion bringt, anschließend die Phasen trennt und die Säurephase durch Entspannungsverdampfung gegebenenfalls unter gleichzeitiger Wärmezufuhr von mindestens 5 % des Wassers destillativ befreit und nach Zugabe von 50 bis 100 Gew.-%iger Salpetersäure kontinuierlich in die Reaktion zurückführt, die in der ersten Stufe abgetrennte organische Phase (hauptsächlich Mononitrotoluol; MNT) unter adiabatischen Bedingungen bei Temperaturen von 20 bis 200°C, vorzugsweise 40 bis 180°C, besonders bevorzugt von 60 bis 170°C, mit einer Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 Gew.-% aus Nitrotoluol-Isomeren und zum Rest aus Nebenprodukten bestehen, zur Reaktion bringt, wobei das Molverhältnis von Salpetersäure zu Mononitrotoluol mindestens 0,7:1 und höchstens 1,2:1 beträgt, anschließend die Phasen trennt und die Säurephase aus der zweiten Stufe durch Entspannungsverdampfung gegebenenfalls unter gleichzeitiger Wärmezufuhr von mindestens 5 % des Wassers destillativ befreit und nach Zugabe von 50 bis 100 Gew.-%iger Salpetersäure kontinuierlich in die Reaktion zurückführt und den Brüden bei der Aufkonzentrierung der zweiten Absäure Mononitrotoluol aus der ersten Stufe zuführt.

## Claims

1. Two-step process for the continuous production of isomeric mixtures of dinitrotoluene by nitration of toluene, characterised in that in the first step, toluene and nitrating acid consisting of (i) 80 to 100 wt.% of inorganic constituents, which are composed substantially of 60 to 90 wt.% sulphuric acid, 1 to 20 wt.% nitric acid and at least 5 wt.% water and (ii) 0 to 20 wt.% organic constituents consisting to the extent of 70 to 100 wt.% of isomers of nitrotoluene, the remainder being by-products, are reacted adiabatically in a continuously operated reactor at temperatures of from 0°C to 140°C, preferably from 20°C to 140°C, a molar ratio of nitric acid to toluene of at least 0.7:1 and at most 1.2:1 being established, the phases are then separated and the acid phase is freed from at least 5% of the water by distillation by the method of flash evaporation optionally with simultaneous input of heat and, after the addition of 50 to 100 wt.% nitric acid, is returned continuously to the reaction, the organic phase separated off in the first step (mainly mononitrotoluene: MNT) is reacted under adiabatic conditions at temperatures of from 20°C to 200°C, preferably from 40°C to 180°C, particularly preferably from 60°C to 170°C, with a nitrating acid consisting of (i) 80 to 100 wt.% of inorganic constituents, which are composed substantially of 60 to 90 wt.% sulphuric acid, 1 to 20 wt.% nitric acid and at least 5 wt.% water and (ii) 0 to 20 wt.% organic constituents consisting to the extent of 70 to 100 wt.% of isomers of nitrotoluene, the remainder being by-products, the molar ratio of nitric acid to mononitrotoluene being at least 0.7:1 and at most 1,2:1, the phases are then separated and the acid phase from the second step is freed from at least 5% of the water by distillation by the method of flash evaporation optionally with simultaneous input of heat and, after the addition of 50 to 100 wt.% nitric acid, is returned continuously to the reaction and mononitrotoluene from the first step is added to the vapours during the concentration of the second spent acid.

## Revendications

1. Procédé à deux stades opératoires pour la préparation en continu de mélanges de dinitrotoluènes isomères par nitration du toluène, caractérisé en ce que, au premier stade opératoire, on fait réagir le toluène et un mélange sulfonitrique consistant en (i) 80 à 100 % en poids de composants inorganiques constitués eux-mêmes essentiellement de 60 à 90 % en poids d'acide sulfurique, 1 à 20 % en poids d'acide nitrique ct au moins 5 % en poids d'eau, et (ii) 0 à 20 % en poids de composants organiques constitués eux-mêmes pour 70 à 100 % en poids, de nitrotoluènes isomères et, pour le solde, de produits d'accompagnement, dans un réacteur opérant en continu, dans des conditions adiabatiques, avec réglage d'un rapport moléculaire acide nitrique/toluène d'au moins 0,7 : 1 et de 1,2 : 1 au maximum, à des températures de 0 à 140°C, de préférence de 20 à 140°C, on sépare ensuite les phases et on débarrasse la phase acide par vaporisation par détente le cas échéant avec apport simultané de chaleur d'au moins 5 % de l'eau par distillation et, après addition d'un acide nitrique à une concentration de 50 à 100% en poids, on la recycle en continu dans la réaction, on fait réagir la phase organique séparée au premier stade (principalement le mononitrotoluène; MNT) dans des conditions adiabatiques à des températures de 20 à 200°C, de préférence de 40 à 180°C et plus spécialement de 60 à 170°C, avec un mélange sulfonitrique consistant en (i) 80 à 100 % en poids de composants inorganiques constitués eux-mêmes essentiellement de 60 à 90 % en poids d'acide sulfurique, 1 à 20 % en poids d'acide nitrique et au moins 5 % en poids d'eau, et (ii) 0 à 20 % en poids de composants organiques constitués eux-mêmes pour 70 à 100 % en poids de nitrotoluènes isomères et, pour le solde, de produits d'accompagnement, à un rapport molaire acide nitrique/mononitrotoluène qui est d'au moins 0,7 : 1 et au maximum de 1,2 : 1, on sépare ensuite les phases et on débarrasse la phase acide du deuxième stade, par vaporisation par détente, le cas échéant avec apport de chaleur simultanée, d'au moins 5 % de l'eau par distillation et après admission d'un complément d'acide nitrique à une concentration de 50 à 100 % en poids, on la recycle en continu dans la réaction, et on envoie du mononitrotoluène du premier stade dans les vapeurs de la reconcentration du deuxième acide résiduaire.
